# FASCICULE DE BREVET EUROPEEN

(11) **EP 4 511 670 B1**
(45) Date de publication et mention de la délivrance du brevet: **03.06.2026**
(21) Numéro de dépôt: 23720618.0
(22) Date de dépôt: 20.04.2023
(51) Int. Cl.: G01R 33/56, G01R 33/565, G01R 33/483, G01R 33/567

(54) **PROCEDE ET SYSTEME D'ACQUISITION ET DE RECONSTRUCTION D'IMAGES D'UN COEUR EN RESPIRATION LIBRE**
VERFAHREN UND SYSTEM ZUR ERFASSUNG UND REKONSTRUKTION VON BILDERN EINES HERZENS WÄHREND DES FREIATMENS
METHOD AND SYSTEM FOR ACQUIRING AND RECONSTRUCTING IMAGES OF A HEART DURING FREE-BREATHING

(30) Priorité: 22.04.2022 FR 2203782
(43) Date de publication de la demande: 26.02.2025
(73) Titulaire: UNIVERSITE DE BORDEAUX, 33000 Bordeaux (FR); Centre Hospitalier Universitaire de Bordeaux, 33404 Talence Cedex (FR); FONDATION BORDEAUX UNIVERSITÉ, 33000 Bordeaux (FR); Centre Hospitalier Universitaire Vaudois, 1011 Lausanne (CH)
(72) Inventeur: STUBER, Matthias, 1032 Romanel-sur-Lausanne (CH); BUSTIN, Aurélien, 33000 Bordeaux (FR); COCHET, Hubert, 33000 Bordeaux (FR)
(74) Mandataire: Oak & Fox
(86) Numéro de dépôt international: PCT/EP2023/060330
(87) Numéro de publication internationale: WO 2023/203151

(56) Documents cités:
- GB-A- 2 572 358
- GINAMI GIULIA ET AL: "3D whole-heart phase sensitive inversion recovery CMR for simultaneous black-blood late gadolinium enhancement and bright-blood coronary CMR angiography", vol. 19, no. 1, 27 November 2017 (2017-11-27), XP093002080, Retrieved from the Internet <URL:https://jcmr-online.biomedcentral.com/counter/pdf/10.1186/s12968-017-0405-z.pdf> DOI: 10.1186/s12968-017-0405-z
- HOLTACKERS ROBERT J. ET AL: "Dark-blood late gadolinium enhancement cardiovascular magnetic resonance for improved detection of subendocardial scar: a review of current techniques", JOURNAL OF CARDIOVASCULAR MAGNETIC RESONANCE, vol. 23, no. 1, 22 July 2021 (2021-07-22), pages 1 - 18, XP055982850, DOI: 10.1186/s12968-021-00777-6
- ZEILINGER MARTIN GEORG ET AL: "Non-rigid motion-corrected free-breathing 3D myocardial Dixon LGE imaging in a clinical setting", EUROPEAN RADIOLOGY, SPRINGER BERLIN HEIDELBERG, BERLIN/HEIDELBERG, vol. 32, no. 7, 20 February 2022 (2022-02-20), pages 4340 - 4351, XP037886329, DOI: 10.1007/S00330-022-08560-6
- KELLMAN PETER ET AL: "Bright-blood and dark-blood phase sensitive inversion recovery late gadolinium enhancement and T1 and T2 maps in a single free-breathing scan: an all-in-one approach", vol. 23, no. 1, 1 December 2021 (2021-12-01), XP093002076, Retrieved from the Internet <URL:https://link.springer.com/content/pdf/10.1186/s12968-021-00823-3.pdf> DOI: 10.1186/s12968-021-00823-3
- AURELIEN BUSTIN ET AL: "Improved Myocardial Scar Visualization with Two-minute Free-breathing Joint Bright- and Black-blood Late Gadolinium Enhancement Imaging", PROCEEDINGS OF THE JOINT ANNUAL MEETING ISMRM-ESMRMB 2022 & ISMRT ANNUAL MEETING, LONDON, UK, 07-12 MAY 2022, ISMRM, 2030 ADDISON STREET, 7TH FLOOR, BERKELEY, CA 94704 USA, no. 271, 22 April 2022 (2022-04-22), XP040726819

## Description

### Domaine de l'invention

L'invention concerne le domaine de l'imagerie par résonance magnétique, notamment, l'imagerie par résonance magnétique cardiaque. Elle concerne plus particulièrement l'imagerie par résonance magnétique ou IRM par rehaussement tardif du gadolinium ou LGE, en référence à l'expression anglo-saxonne « Late Gadolinium Enhancement ». Elle s'applique à l'imagerie cardiaque, mais également à l'angiographie. Le domaine de l'invention se rapporte plus particulièrement aux procédés et dispositifs d'imagerie pour détecter et localiser une cicatrice dans le tissu myocardique.

### État de la technique

La technique de référence pour l'évaluation de la formation de cicatrices régionales et de la fibrose myocardique est l'imagerie par rehaussement tardif du gadolinium en sang blanc ou BR-LGE (pour BRight-Blood LGE). Dans ce type d'imagerie, on provoque l'annulation du signal myocardique viable à l'aide d'impulsions d'inversion-récupération ce qui permet de visualiser des cicatrices avec un contraste élevé entre le tissu myocardique sain et les cicatrices. Cependant, pour les petites cicatrices ou les cicatrices myocardiques adjacentes aux cavités sanguines du cœur, l'intensité élevée du signal issu du sang empêche la visualisation claire et la délimitation précise des cicatrices, en particulier des cicatrices subendocardiques. Afin de contourner ce problème, des techniques d'imagerie LGE en sang noir (BL-LGE, pour BLack-blood LGE) ont été proposées. Elles permettent d'annuler simultanément les signaux du myocarde sain et du sang fournissant ainsi un contraste élevé à la fois entre les cicatrices et entre le sang et entre les cicatrices et le myocarde sain.

L'annulation du signal du sang, pour obtenir un contraste "sang noir", est obtenue en appliquant, à une zone à imager d'un patient, une séquence radiofréquence (RF) d'inversion-récupération avec une impulsion de 180° suivie par un module préparatoire et un module de lecture. Le temps entre le module préparatoire qui survient juste après l'émission d'un pulse radiofréquence d'inversion de l'aimantation et la séquence de lecture est appelé temps d'inversion TI.

On cherche à acquérir les signaux pour générer l'image de la zone à imager de manière à localiser et à détecter une cicatrice présente dans les tissus myocardiques.

Du document Ginami Giulia et AL : "3D Whole-heat phase sensitive inversion recovery CMR for simultaneous black-blood late gadolinium enhancement and bright-blood coronary CMR angiography", Journal of Cardiovascular Magnetic Resonance, vol 19, no 1, 27 novembre 2017, on connaît une solution en acquisition sang noir- sang blanc.

Un inconvénient des solutions de l'art antérieur dite en acquisition sang noir - sang blanc est qu'elles nécessitent un temps d'acquisition important du fait de l'acquisition en 3D impliquant de nombreux calculs. Le patient est dans ces conditions en respiration libre. Une conséquence est de devoir appliquer des algorithmes de correction d'images requérant de grandes ressources de calculs impliquant des temps de calcul rallongeant la séquence d'acquisition et une difficulté de les appliquer en temps réel. Or lorsque le temps d'acquisition est rallongé, la variabilité des conséquences de la respiration aboutit en la production d'artefacts de traitement d'images altérant la lisibilité de ces dernières. Ces artefacts accentuent la difficulté de reconstruire des images nettes et précises afin de localiser et détecter la cicatrice. Par ailleurs, les longues acquisitions en IRM sont inconfortables pour le patient. Une durée de 10 à 20 min est considérée comme une durée très longue et il est difficile pour le patient de se maintenir au sein de l'IRM sans effectuer de mouvement.

Aujourd'hui, ces séquences nécessitent un temps d'acquisition de plus de 15 min. Les images acquises ne sont pas satisfaisantes dans de nombreux cas, car les artefacts d'une image extraite d'un plan de coupe de l'image 3D aboutit à des cas dans lesquels il est impossible de discriminer la présence d'une cicatrice potentielle de présence du sang située à proximité du muscle. En effet, dans certains cas, la cicatrice est tellement proche du sang, on la nomme sous endocardique, qu'il est difficile de savoir si c'est une cicatrice, du sang ou un artefact de l'image. Un problème est de fonder une décision clinique aboutissant à une erreur de diagnostic sur la présence d'une cicatrice ou non.

Une autre solution consiste à faire une acquisition rapide en apnée, mais cette solution entraîne la nécessité de réduire le temps d'acquisition de quelques secondes à quelques minutes et implique souvent de devoir réduire la zone à imager par exemple en se limitant à une portion 3D.

Or il est néanmoins nécessaire d'obtenir un minimum d'images pour reconstruire une portion 3D. Un problème est que les battements cardiaques influencent également la mesure et impliquent de réaliser des acquisitions synchronisées sur le battement cardiaque. Cette contrainte impose de réduire le nombre d'acquisitions potentiel sur une portion de temps donnée. En effet, il est nécessaire de séquencer les acquisitions sur le rythme cardiaque, il est donc nécessaire d'étaler ces acquisitions sur un nombre minimum de battements. Les acquisitions en apnées contraignent fortement la capacité d'acquisition.

Enfin, il existe des méthodes d'acquisition 2D, mais ces dernières ne permettent pas d'obtenir une résolution et un contraste suffisant pour détecter et localiser les cicatrices. Il serait nécessaire de réaliser plusieurs acquisitions 2D d'une même zone pour désambiguïser certains cas, mais les variations de la respiration et les battements cardiaques au cours du temps ne permettent pas de réaliser des images 2D sur des plans de coupe de l'organe identiques au cours du temps.

### Résumé de l'invention et Avantages

Selon un premier aspect l'invention concerne un procédé de reconstruction d'une image d'un cœur d'un patient à partir d'un dispositif d'imagerie par résonance magnétique générant un champ magnétique comprenant :
- Une première phase de génération d'images comportant :
   ■ Acquisition de l'activité électrique du cœur du patient ;
   ■ Génération d'un premier signal radiofréquence d'inversion de 180° pour faire basculer une aimantation longitudinale de tissus d'une zone imagée, ledit premier signal radiofréquence d'inversion étant généré entre deux complexes QRS de l'activité électrique acquise, dite première phase inter-battement ;
   ■ Génération d'une première préparation d'aimantation comportant un ensemble de pulses consécutivement à la génération du premier signal radio fréquence d'inversion dans la même première phase inter-battement ;
   ■ Acquisition d'une première image 2D par une mesure d'aimantation de la zone imagée, ladite acquisition étant réalisée après une première durée prédéfinie consécutivement à la génération de la première préparation d'aimantation, ladite acquisition étant synchronisée avec le rythme cardiaque à un premier marqueur temporel de la même phase inter-battement ;
   ■ Génération d'une seconde préparation d'aimantation comportant un ensemble de pulses dans une seconde phase inter-battement succédant à la première phase inter-battement ;
   ■ Acquisition d'une seconde image 2D par une mesure d'aimantation de la zone imagée, ladite acquisition étant réalisée après une seconde durée prédéfinie consécutivement à la génération de la seconde préparation d'aimantation, ladite acquisition étant synchronisée avec l'acquisition d'un rythme cardiaque au même premier marqueur temporel de la seconde phase inter-battement que le premier marqueur de la première phase inter-battement ;
- Répétition de la première phase pour générer une pluralité de premières images et une pluralité de secondes images pour une pluralité de plans de coupes du cœur dans des phases inter-battements succédant la première et la seconde phases inter-battements, chaque paire de première image et de seconde image étant associée à un plan de coupe ;

Génération d'un ensemble de troisièmes images 2D pour chaque plan de coupe, chaque troisième image correspondant à une première combinaison réalisée entre chaque paire de premières images fusionnées et de secondes images fusionnées pour chaque plan de coupe.

Cet aspect de l'invention concerne le cas où une image par plan de coupe est acquise. Cette possibilité est offerte pour des acquisitions rapides avec nécessitant une trentaine de battements pour couvrir une quinzaine de plans de coupe avec une image sang noir et une image sang blanc par coupe. Toutefois, cette séquence peut être améliorée par une acquisition en respiration libre en acquérant plusieurs images par plan de coupe pour le sang noir d'une part et pour le sang blanc d'autre part.

Selon un autre aspect, l'invention concerne un procédé de reconstruction d'une image d'un cœur d'un patient à partir d'un dispositif d'imagerie par résonance magnétique générant un champ magnétique comprenant :
- Une première phase de génération d'images comportant :
   ■ Acquisition de l'activité électrique du cœur du patient ;
   ■ Génération d'un premier module radiofréquence d'inversion de 180° de façon à faire basculer une aimantation longitudinale d'une zone imagée, ledit premier signal radiofréquence d'inversion étant généré entre deux complexes QRS de l'activité électrique acquise, dite première phase inter-battement ;
   ■ Génération d'une première préparation d'aimantation comportant un ensemble de pulses consécutivement à la génération du premier module radiofréquence d'inversion dans la même première phase inter-battement ;
   ■ Acquisition d'une première image 2D par une mesure d'aimantation de la zone imagée, ladite acquisition étant réalisée après une première durée prédéfinie consécutivement à la génération de la première préparation d'aimantation, ladite acquisition étant synchronisée avec le rythme cardiaque à un premier marqueur temporel de la même phase inter-battement ;
   ■ Génération d'une seconde préparation d'aimantation comportant un ensemble de pulses dans une seconde phase inter-battement succédant à la première phase inter-battement ;
   ■ Acquisition d'une seconde image 2D par une mesure d'aimantation de la zone imagée, ladite acquisition étant réalisée après une seconde durée prédéfinie consécutivement à la génération de la seconde préparation d'aimantation, ladite acquisition étant synchronisée avec l'acquisition d'un rythme cardiaque au même premier marqueur temporel de la seconde phase inter-battement que le premier marqueur de la première phase inter-battement ;
- Répétition de la première phase pour générer une pluralité de premières images et une pluralité de secondes images pour une pluralité de plans de coupes du cœur dans des phases inter-battements succédant la première et le seconde phases inter-battements, chaque paire de première image et de seconde image étant associée à un plan de coupe ;
- Répétition de la première phase pour générer une pluralité de premières images et de secondes images d'un même plan de coupe du cœur dans des phases inter-battements succédant la première et la seconde phases inter-battements.

Autrement dit, le procédé comprend la répétition de la première phase, pour générer un sous-ensemble de premières images et un sous-ensemble de secondes images de chaque plan de coupe d'une pluralité de plans de coupes du cœur, dans des phases inter-battements succédant la première et la seconde phases inter-battements.

Le procédé comprend également :
- Application d'un algorithme de recalage non rigide à chaque sous-ensemble de premières images d'un même plan de coupe pour recaler lesdites premières images entre elles ;
- Application d'un algorithme de recalage non rigide à chaque sous-ensemble de secondes images d'un même plan de coupe pour recaler lesdites secondes images entre elles ;
- Fusion d'une part des premières images recalées entre elles pour produire une première image fusionnée et d'autre part des secondes images recalées entre elles pour produire une seconde image fusionnée ;
- Génération d'un ensemble de troisièmes images 2D pour chaque plan de coupe, chaque troisième image correspondant à une première combinaison réalisée entre chaque paire de premières images fusionnées et de secondes images fusionnées pour chaque plan de coupe.

Un avantage est de permettre de produire des acquisitions en respiration libres de courtes durées ne nécessitant pas les temps de calcul d'une acquisition 3D.

Avantageusement, le procédé comprend les étapes suivantes :
- fusion, pour chacun des plans de coupe, d'une part des premières images recalées entre elles pour produire une première image fusionnée et d'autre part des secondes images recalées entre elles pour produire une seconde image fusionnée ;
- génération, pour lesdits plans de coupe, d'un ensemble de troisièmes images 2D, chaque troisième image correspondant à une première combinaison réalisée entre la première image fusionnée et la seconde image fusionnée produites pour un des plans de coupe.

Avantageusement :
- pour chaque plan de coupe, on applique un algorithme de recalage non rigide aux premières images du plan de coupe pour recaler lesdites premières images du sous-ensemble de premières images entre elles ;
- pour chaque plan de coupe, on applique un algorithme de recalage non rigide aux premières images du plan de coupe pour recaler lesdites premières images du sous-ensemble de premières images entre elles.

Selon l'invention, la première durée étant déterminée de sorte que les premières images acquises sont des images ayant un premier contraste permettant d'afficher des images en sang noir et la seconde durée étant déterminée de sorte que les secondes images acquises sont des images ayant un second contraste permettant d'afficher des images en sang blanc.

Selon un mode de réalisation, l'étape de recalage est suivie des étapes de :
- Moyennage des premières images acquises recalées pour produire une première image fusionnée pour chaque plan de coupe ;
- Moyennage des secondes images acquises recalées pour produire une première image fusionnée pour chaque plan de coupe.

Un avantage est d'augmenter le rapport signal sur bruit, également appelé SNR.

Selon un mode de réalisation, la première durée est le temps d'inversion en inversion-récupération.

Avantageusement, la première durée est définie de façon que l'aimantation longitudinale du sang et celle du myocarde sain s'annulent au même instant lors de l'acquisition de la première image 2D.

Selon un mode de réalisation, la détermination de la première durée est automatiquement calculée à partir d'un procédé mis en œuvre par ordinateur de calcul d'un temps d'inversion optimal réalisé à partir d'un traitement d'au moins une image acquise à partir d'une préséquence d'IRM.

Ce calcul est par exemple réalisé par le procédé, mis en œuvre par ordinateur, de détermination du temps d'inversion optimal décrit dans la demande de brevet FR2203766. La préséquence d'IRM comprend l'acquisition d'une pluralité d'images IRM par des séquences d'acquisition présentant des temps d'inversion respectifs distincts. Le temps d'inversion est calculé à partir de ces images.

Selon un mode de réalisation, le procédé comprend une étape de colorisation des pixels de chaque première image fusionnée ayant une luminance supérieure à un seuil donné, l'étape de colorisation étant réalisée préalablement à la première combinaison. Dans le cas d'exemple les images colorées correspondent aux images en sang noir.

Un avantage est de permettre de localiser et identifier une cicatrice à proximité du myocarde et de volume(s) de sang de manière sûre, c'est-à-dire sans ambiguïté.

Selon un mode de réalisation, la première combinaison est une superposition de la première image sur la seconde image fusionnée. C'est-à-dire une superposition de l'image en sang noir colorée sur l'image en sang blanc colorée.

Dans une réalisation particulière, la première image est la première image fusionnée.

Selon un mode de réalisation, chaque nouvelle génération d'une pluralité de premières images et de secondes images d'un même plan de coupe est réalisée après chaque répétition de la première phase pour générer une pluralité de premières images et une pluralité de secondes images pour une pluralité de plans de coupes du cœur.

Selon un mode de réalisation, chaque génération d'une nouvelle première image et d'une nouvelle seconde image dans un nouveau plan de coupe du cœur est réalisée après l'ensemble des répétitions de la première phase pour générer une pluralité de premières images et de secondes images d'un précédent plan de coupe du cœur.

Un avantage est de réaliser toutes les images d'un même plan coupe dans un intervalle de temps court pour éviter les dérives de décalages d'images dans un même plan de coupe lorsqu'elles sont entrelacées entre après l'acquisition de différentes images d'autres plans de coupes.

Selon un mode de réalisation, le procédé comporte :
■ un affichage d'un ensemble de premières images fusionnées pour chaque plan de coupe ;
■ un affichage d'un ensemble de secondes images fusionnées pour chaque plan de coupe ;
■ un affichage d'un ensemble de troisièmes images 2D pour chaque plan de coupe ;
■ chaque première image d'un plan de coupe donné étant affichée au voisinage immédiat de la seconde image et de la troisième image du même plan de coupe donné.

Avantageusement, la première image affichée et la deuxième image affichée pour un plan de coupe sont respectivement la première image fusionnée et la deuxième image fusionnée.

Selon un mode de réalisation, la première image fusionnée pour chaque plan de coupe correspond à une image d'une coupe du cœur en sang noir, la seconde image fusionnée pour chaque plan de coupe correspond à une image d'une coupe du cœur en sang blanc, la troisième image pour chaque plan de coupe correspondant à une image d'une coupe de l'organe sur laquelle une cicatrice le cas échéant est affichée en couleur.

Selon un mode de réalisation, la première et la seconde préparation d'aimantation est une pondération T1 en T1rho, ledit procédé étant réalisé lors du rehaussement tardif après injection de Gadolinium.

Selon un mode de réalisation, la première préparation d'aimantation est une préparation de type T1rho, ladite préparation T1rho étant définie par l'enchaînement d'une configuration de l'aimant défini ainsi : 90x-SLy-180y-SL-y-SLy-180-y-SL-y-90-x.

Selon un autre exemple de préparation de type T1rho, une autre configuration d'aimantation peut être par exemple 90x-Sly-90-x.

Selon un mode de réalisation, la première préparation d'aimantation est une préparation de type T2prep ou une préparation de type MTC.

Selon un mode de réalisation, la génération d'une seconde préparation d'aimantation comportant un ensemble de pulses dans une phase inter-battement est succédé à une étape de filtre des images acquises correspondant à des zones graisseuses de l'organe imagé.

Selon un mode de réalisation, les premières images et les secondes images sont acquises successivement de manière synchronisée avec l'activité électrique du cœur entre deux complexes QRS pendant plusieurs minutes en respiration libre lors d'un unique examen.

Selon un mode de réalisation, les premières images et les secondes images sont acquises sur un ensemble de 8 à 20 plans de coupe de l'organe, la phase de réitération des acquisitions d'une pluralité de premières images et de secondes images dans un même plan de coupe permettant d'acquérir entre 2 et 10 images de premières images et entre 2 et 10 images de secondes images par plan de coupe.

Selon un autre aspect, l'invention concerne un système d'imagerie par résonnance magnétique comprenant un générateur de champ magnétique et un dispositif radiofréquence, un électrocardiographe et un dispositif de traitement, un afficheur pour afficher les images générées, ledit système étant configuré pour mettre en œuvre le procédé de l'invention.

Un avantage est de permettre de générer des images dans lesquelles il est possible pour un radiologue de localiser et détecter la cicatrice des tissus du myocarde de manière fiable.

Un avantage est de permettre d'afficher une zone d'intérêt telle qu'une cicatrice en couleur.

Un autre avantage est de permettre d'acquérir rapidement des images en respiration libre ne contraignant pas notamment pour le patient la nécessité de réaliser l'imagerie en apnée.

Par ailleurs, le procédé selon l'invention est simple, rapide et peu coûteux en termes de calculs. Il est facilement intégrable à un dispositif IRM existant sans modification.

Un avantage est que pour chaque coupe donnée du cœur, on collecte une pluralité d'images ce qui permet de réduire le bruit, ou d'augmenter le rapport signal sur bruit et réduire les artefacts résiduels.

Un avantage de l'utilisation d'un algorithme non rigide est de pallier les artefacts de mouvement liés à la variation de la respiration. La mise en œuvre d'un tel algorithme permet d'augmenter le signal sur bruit.

Enfin, l'acquisition en 2D permet une acquisition plus rapide et plus robuste qu'une acquisition en 3D.

### Brève description des figures

D'autres caractéristiques et avantages de l'invention ressortiront à la lecture de la description détaillée qui suit, en référence aux figures annexées, qui illustrent :

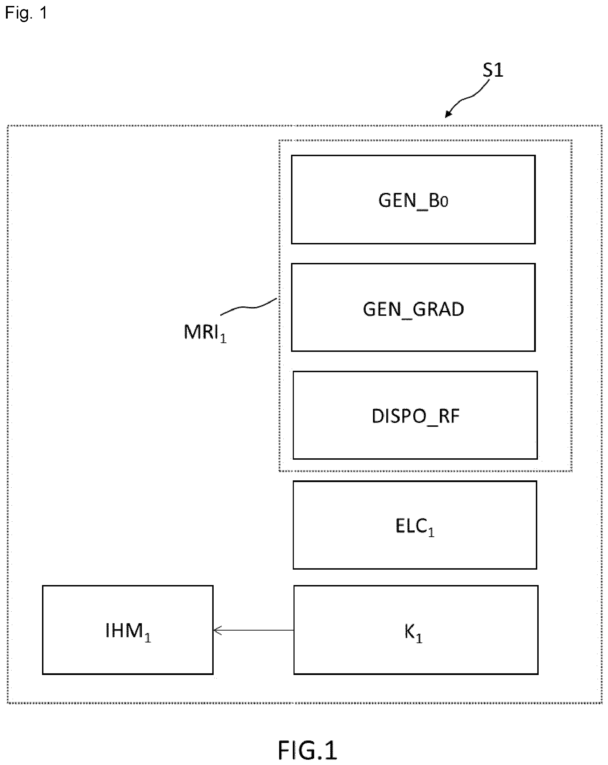

Fig. 1 : un exemple de réalisation d'un système de l'invention comportant un dispositif IRM, un électrocardiographe, un calcul pour réaliser les opérations de traitements des images acquises et une interface homme machine pour visualiser les images ;
Fig. 2 : une représentation schématique d'une portion de la séquence générée par un dispositif IRM sur deux battements cardiaques,
Fig. 3 : une représentation schématique d'une phase d'acquisition IRM en sang noir et sang blanc réalisée sur une pluralité de battements cardiaques,
Fig. 4 : une représentation schématique d'un cœur en 3D illustrant les différents plans de coupes et les images acquises dans chaque plan de coupe,
Fig. 5 : un ordinogramme des étapes du procédé selon un mode de réalisation de l'invention.

### Description invention

### Système IRM

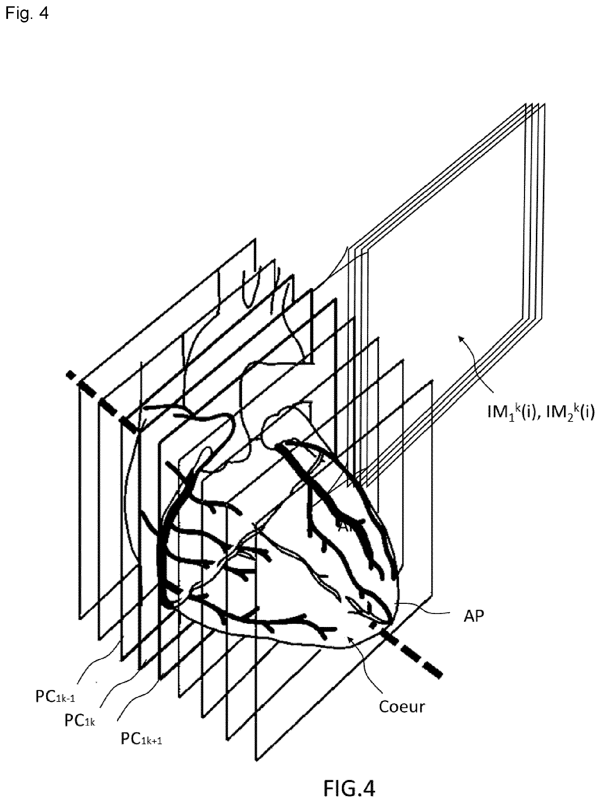

La figure 4 représente schématiquement un système d'imagerie S₁ comportant un dispositif par résonance magnétique MRI₁ selon l'invention comprenant un ensemble d'équipements d'excitation et de mesure, un calculateur K₁ pour effectuer les traitements sur les images acquises et un afficheur IHM₁ pour afficher les images générées.

De façon connue en soit, l'ensemble d'équipements d'excitation et de mesure comprennent un dispositif d'imagerie IRM comprenant un générateur de champ magnétique statique GEN_B₀ comprenant un aimant principal de polarisation, un générateur de gradient GEN_GRAD, et un dispositif radiofréquence DISPO_RF comprenant un générateur de signaux radio fréquences et une antenne radiofréquences.

Le générateur de champ magnétique statique GEN_B₀ comprend un aimant destiné à générer un champ magnétique statique de polarisation sensiblement uniforme dans une zone de polarisation (généralement un tunnel) destinée à comprendre la zone à imager du patient, par exemple le cœur.

Le générateur de gradient GEN_GRAD comprend trois bobines ou solénoïdes de gradient disposées et configurées pour faire varier l'intensité du champ magnétique dans la zone de polarisation selon des axes respectifs orthogonaux classiquement notés x, y et z fixes par rapport à la zone de polarisation. Le choix des intensités circulant dans ces bobines permet de sélectionner, parmi plusieurs possibles, une épaisseur et un plan de coupe dans lequel va être mesurée l'aimantation de la zone à imager du patient reçu dans la zone de polarisation.

Le dispositif radiofréquence GEN_RF comprend des bobines ou solénoïdes et est apte à générer des séquences d'acquisition IRM comprenant des séquences de préparation de l'aimantation de la zone à imager et des séquences de lecture de signaux issus de la zone à imager. Chaque séquence d'acquisition comprend au moins un pulse radiofréquence de fréquence, de forme, de durée, de phase, d'amplitude prédéterminées et commandables.

Le module préparatoire, également appelé préparation ou phase de préparation est configuré pour exciter, c'est-à-dire modifier la direction de l'aimantation des tissus de zone à imager, c'est-à-dire moduler leur aimantation. Elle est notée TAprep ou TBprep ou encore T1rho dans la suite de la description.

Le module de lecture est configuré pour mesurer l'aimantation de la zone à imager résultant de la séquence de préparation. Le module de lecture est également appelé module d'acquisition ou phase de lecture ou fenêtre de lecture.

Autrement dit, les images sont acquises par des mesures d'aimantation de la zone à imager.

L'ensemble comprend avantageusement un électrocardiographe ELC₁ destiné à acquérir un électrocardiogramme ECG₁ du patient.

Le système d'imagerie comprend un dispositif de traitement K₁ configuré pour réaliser des calculs notamment de recalage d'images et de moyennage desdites images. Le dispositif de traitement permet en outre éventuellement de colorer les images.

Le système d'imagerie S1 comprend en outre un afficheur pour afficher les images bidimensionnelles en niveaux de gris et/ou en couleur de la zone à imager à partir des signaux mesurés par le générateur de champ magnétique RF et traités par le dispositif de traitement.

Selon un mode de réalisation, l'image reconstruite est une image matricielle en niveau de gris et en couleur. Elle comprend un ensemble de pixels caractérisé chacun par une intensité I susceptible de prendre un ensemble de M valeurs (M étant un nombre entier fini supérieur à 1) correspondant à M niveaux de gris allant de 0 et M-1. Par exemple, cette valeur peut prendre 256 valeurs comprises entre 0 et 255. Cette valeur peut être plus élevée dans d'autres modes de réalisations. Selon un mode, les images peuvent être colorées en partie pour une sélection de pixels ayant une luminance supérieure à un seuil donné.

Des représentations des images générées par le dispositif de traitement K₁ sont destinées à être affichées sur un écran de l'interface homme-machine IHM1 du système d'imagerie S₁.

### Séquence d'acquisition

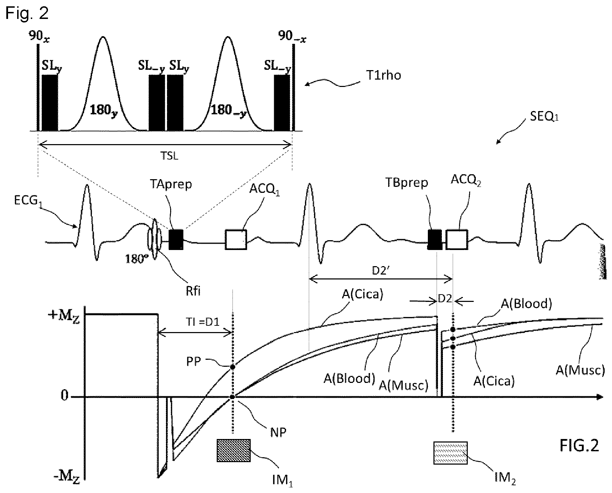

La figure 2 représente un exemple d'une portion de séquence SEQ₁ d'acquisition IRM en sang noir en sang blanc d'une image du myocarde ainsi que l'activité électrique ECG₁ représentée sur deux battements consécutifs. Un électrocardiogramme ECG₁ acquis par l'électrocardiographe ECL₁ lors de deux battements cardiaques ainsi que la séquence d'acquisition SEQ₁ appliquée à la zone à imager sont superposés afin de mieux représenter les caractéristiques temporelles liées entre elles de chacune de ces courbes. L'acquisition de l'ECG est une étape notée ACQ₀. La partie basse de la figure 2 représente la variation de l'aimantation longitudinale Mz des tissues de la zone à imager en fonction du temps t ainsi que deux images IM₁ et IM₂ reconstruites à partir de signaux mesurés lors de la séquence.

Préférentiellement, l'invention permet de synchroniser les séquences d'excitation et acquisition IRM sur l'électrocardiogramme ECG₁. Cette synchronisation permet d'une part de réaliser des mesures à des instants où le cœur est le plus stable et d'autre part de faire des mesures à des instants équivalents entre deux mêmes positions du cœur afin de comparer des images d'une même coupe. Une manière de synchroniser ces excitations et acquisitions et de trigger l'appareil de mesure sur le complexe QRS.

Dans la suite de la description, la première image IM₁ se référera à une image acquise en sang noir et la seconde image IM₂ se référera à une image acquise en sang blanc. Selon un exemple, pendant toute la séquence, à partir du premier battement pendant lequel les acquisitions d'images sont réalisées, les battements impairs sont utilisés pour acquérir les images en sang noir et les battements pairs sont utilisés pour acquérir les images en sang blanc. Dans la suite de la description, cet exemple sera plus détaillé. D'autres configurations sont toutefois envisageables pour exécuter le procédé de l'invention, par exemple associer les battements impairs à l'acquisition d'images en sang blanc et les battements pairs à l'acquisition en sang noir.

L'invention s'applique également dans le cas où les images en sang blanc seraient acquises avant les images en sang noir. L'invention s'applique également dans le cas où les images acquises en sang noir et en sang blanc ne seraient pas immédiatement acquises sur deux battements consécutifs, mais par exemple espacés d'un ou plusieurs battement(s) sans mesure. Toutefois, un intérêt d'acquérir ces images sur deux battements consécutifs est de minimiser le temps d'acquisition et donc de minimiser les effets de la respiration et donc les artefacts d'images sur une échelle de temps de deux battements.

Pour l'acquisition IRM en sang noir et en sang blanc, un produit de contraste à base de Gadolinium est avantageusement injecté par voie intraveineuse chez le patient, de 10 à 15 minutes avant l'application des séquences d'acquisition de façon à obtenir des images présentant un contraste maximal entre les cicatrices et les tissus sains et le sang. Au niveau du cœur, le contraste est rapidement éliminé du myocarde sain, pauvre en tissu interstitiel, mais s'accumule de façon prolongée dans les cicatrices myocardiques. L'effet du gadolinium a pour effet de raccourcir le temps de relaxation T1 des tissus où il s'accumule. La relaxation de l'aimantation des cicatrices suite à une impulsion de l'aimantation est ainsi plus rapide que celle du sang et du myocarde sain.

Dans un premier temps, on cherche à générer des images présentant un contraste en sang noir. Dans une image de ce type, l'intensité des pixels correspondant au sang et au muscle sain est nulle (pixels noirs) ou sensiblement nulle du fait qu'elle est acquise lorsque l'aimantation longitudinale du sang est nulle.

Afin de générer une telle image IM₁, le dispositif DISPO_RF génère une séquence d'acquisition d'inversion-récupération.

Cette séquence d'acquisition comprend une impulsion d'inversion de 180° notée Rfi sur la figure 2, qui fait basculer l'aimantation longitudinale des tissus de la zone imagée dans la direction opposée, c'est-à-dire qui inverse l'aimantation longitudinale. Sur la figure 2, on constate que l'aimantation de la zone à imager passe de Mz à -Mz sous l'effet de l'impulsion d'inversion Rfi. Du fait de la relaxation longitudinale, l'aimantation longitudinale des différents tissus présents dans la zone à imager croît pour revenir à sa valeur initiale, en passant par la valeur nulle. Naturellement, les vitesses de retournement des aimantations des différents tissus sont différentes.

De façon connue en soi, la séquence de préparation comprend ensuite un module préparatoire, par exemple une préparation adiabatique en T1rho, notée T1rho ou TAprep sur la figure 2 de durée TSL, désignant dans la terminologie anglo-saxonne « Spin Lock Time ».

D'autres préparations, tels qu'une préparation T2prep une préparation en transfert d'aimantation MT peut être utilisée.

On note dans la présente demande que la préparation peut tantôt être défini comme la préparation T1rho ou tantôt être défini comme la préparation T1rho précédée de la séquence d'inversion Rfi à 180°. La seconde image IM2 étant acquise sans pulse d'inversion à 180°, mais uniquement avec une préparation T1rho, la préparation dans ce cas est limitée à la préparation T1rho.

La séquence de préparation (Rfi(180°), T1rho) est configurée de sorte que l'aimantation longitudinale du sang et celle du myocarde sain s'annulent au même instant TI, voir la position du point NP de la courbe A(blood) et A(Musc). Le temps d'inversion TI correspond à une mesure de temps entre l'émission du signal Rf à 180 et le module d'acquisition ACQ₁. L'instant TI correspond à la durée notée D₁ dans la présence demande. En effet, l'invention s'applique également à des cas dans lesquels la mesure de l'aimantation ne serait pas nécessairement calculée au temps d'inversion, mais possiblement à un autre moment en fonction du résultat souhaité.

On note indifféremment une fenêtre d'acquisition ou une fenêtre de lecture pour chaque battement, elles sont notées ACQ₁ pour les fenêtres de lecture se rapportant à l'acquisition en sang noir et ACQ₂ les fenêtres de lecture se rapportant à l'acquisition en sang blanc.

On note que sur la figure 2, à ce même instant TI, l'aimantation longitudinale des cicatrices A(Cica) est nettement supérieure à zéro, en effet le point PP est au-dessus de zéro. En faisant l'acquisition des signaux issus de la zone à imager à cet instant TI, on obtient une image présentant un contraste très élevé entre les pixels correspondant au sang et au myocarde sain qui sont noirs et les cicatrices, globalement blanches.

La séquence d'inversion-récupération comprend ensuite une séquence de lecture ACQ₁ comprenant une impulsion de 90° et un gradient de lecture pour lire l'aimantation transversale de la zone à imager.

Le temps d'inversion TI est la durée séparant l'impulsion de 180° de la fenêtre d'acquisition ACQ₁. Le marqueur temporel de la fenêtre d'acquisition ACQ₁ est imposé en amont dans l'ECG en général en diastole. Il est choisi comme l'instant où les signaux du muscle et du sang s'annulent, c'est-à-dire là où les aimantations longitudinales du sang et du myocarde s'annulent afin de générer l'image présentant le meilleur contraste. Afin d'obtenir le meilleur contraste entre les cicatrices myocardiques et le sang et le myocarde sain, on cherche à faire débuter -le signal Rf d'inversion à un instant antérieur pour provoquer l'annulation des signaux à une durée TI.

La séquence est suivie d'une phase d'acquisition en sang blanc réalisée au battement suivant l'acquisition en sang noir. Dans cette phase, une seconde image IM₂ est acquise. La phase de préparation est notée TBprep sur la figure 2. Selon un mode de réalisation, la préparation TBprep pourrait être différente de celle de la préparation TAprep utilisée pour imager la zone du cœur en sang noir, toutefois il est préférable que les préparations en sang noir TAprep et en sang blanc TBprep soient identiques. L'invention se rapporte donc à d'autres préparations, toutefois dans la suite de la description, la phase d'acquisition en sang blanc est décrite avec une même préparation en sang blanc que la préparation en sang noir, par exemple une préparation T1rho.

La durée D₂ séparant le marqueur temporel de la fenêtre d'acquisition AQ₂, qui est fixée pour chaque battement et le marqueur temporel correspondant à la préparation TBprep en sang blanc peut être ajustée pour optimiser le contraste en sang blanc afin d'imager au mieux les contours du muscle vis-à-vis du sang.

La mesure des gradients d'aimantation est configurée pour une acquisition ACQ₂ de l'image en sang blanc au même marqueur inter-battement que l'image en sang noir. Ainsi, sur la figure 2, la phase de lecture ACQ₂ est située à une durée du complexe QRS précédent identique à la durée entre le QRS du battement précédent la phase de lecture ACQ₁.

Lors de la lecture des aimantations à ce même instant, il est possible d'obtenir une image dans laquelle les pixels des zones de sang sont représentés en blanc, c'est-à-dire avec une forte luminance, et dans laquelle les pixels des tissus du myocarde sont un peu moins lumineux que ceux du sang. Ainsi, il est possible d'obtenir la topologie en 2D de la coupe permettant de visualiser les zones de sang et de myocarde les unes par rapport aux autres.

On comprend qu'en combinant les images IM₁ et IM₂ il sera possible de localiser et détecter la cicatrice présente le cas échéant.

A cet effet, l'invention permet de réaliser des mesures successives de l'enchaînement de ces deux images IM₁ en sang noir et IM₂ en sang blanc sur une pluralité de coupes du cœur pour étudier l'intégralité du cœur et en prenant en compte plusieurs images par coupe afin de rendre plus robuste l'analyse des images.

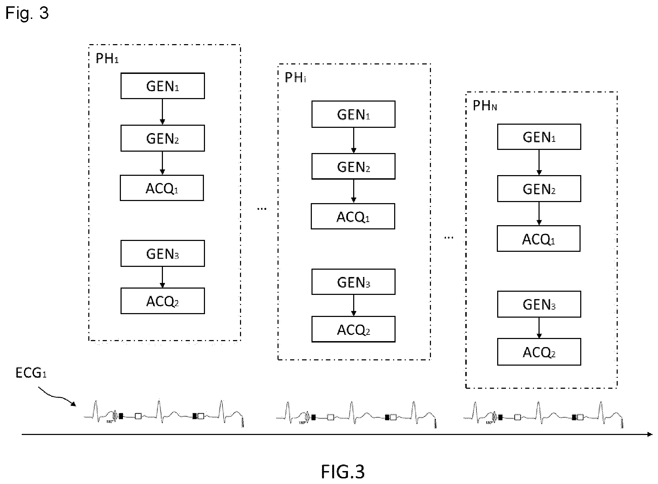

La figure 3 représente un exemple d'enchaînement de ces phases noté Phi, avec i = 1 à N, sur une pluralité de couples de battements consécutifs. Les phases PHi comprennent à chaque fois sur deux battements :
- Premier battement : une génération GEN₁ d'un signal RF d'inversion, une phase de préparation générée et notée GEN₂ et une phase de lecture notée ACQ₁ ;
- Second battement : la génération d'une GEN₃ phase de préparation et une phase de lecture notée ACQ₂.

La figure 4 représente une vue 3D d'un cœur comportant une pluralité de K plans de coupes notés PCₖ, avec k = 1 à K. La figure 4 permet de représenter une pluralité de plans de coupe comprenant chacun une pluralité d'images acquises grâce au procédé de l'invention. Les images du plan de coupe PCₖ sont représentées, les autres ne sont pas représentées sur la figure 4. Les images sont notées respectivement IM₁^{k}(i) pour la i^{ème} image acquise de la coupe PCₖ, avec i = 1 à N.

Selon un mode de réalisation, une première acquisition de l'image du cœur permet d'évaluer le nombre de plans de coupes et donc le nombre de répétitions d'acquisition. Si le cœur est considéré de petite taille, entre 7 et 9 plans de coupes peuvent être définis, si le cœur est de plus grande taille, entre 14 et 20 coupes peuvent être définies. Si le cœur a une taille moyenne, le nombre de coupes peut être défini entre 10 et 13 coupes.

Cette estimation peut être réalisée au début de l'examen, par exemple lors d'une préséquence.

Selon un autre exemple, l'évaluation de la taille du cœur est déterminée automatiquement lors des premières acquisitions du procédé de l'invention, pendant les premiers battements. Cette taille peut automatiquement permettre de réaliser un calcul dynamiquement de manière à acquérir des images selon un nombre donné de coupes.

Selon un exemple d'une séquence complète de 3 min avec un pouls de 60 battements par min, en considérant une configuration de 15 plans de coupe pour l'organe, on aurait en tout 3x60 = 180 images, dont 90 images en sang noir et 90 d'images en sang blanc. Une telle configuration permettait d'acquérir 6 images {IM₁^{k}(1), IM₁^{k}(2), IM₁^{k}(3), IM₁^{k}(4), IM₁^{k}(5), IM₁^{k(6)}} par plan de coupe en sang noir et 6 images acquises {IM₂^{k}(1), IM₂^{k}(2), IM₂^{k}(3), IM₂^{k}(4), IM₂^{k}(5), IM₂^{k}(6)} par plan de coupe en sang noir.

Un avantage du procédé de l'invention est d'être réalisé en respiration libre. Dans cet objectif, il est nécessaire que les images acquises dans une même coupe soient traitées afin qu'elles ne soient pas altérées par les effets de la respiration libre. Le rythme de la respiration est a priori différent du rythme cardiaque. Même si des corrélations existent entre ces deux rythmes, il est possible que la respiration s'accélère alors que le battement cardiaque reste stable ou inversement.

Le problème est que lors de la seconde lecture ACQ₂, la respiration a pu modifier sensiblement la position du cœur, ce qui a pour conséquence que les images acquises à un autre battement de la même coupe peuvent être décalées vis-à-vis d'une image acquise précédemment dans la même coupe.

Selon un mode de réalisation, les images de chaque coupe issue de l'acquisition en sang noir sont recalées à partir d'un algorithme de recalage d'images non rigide. Les images acquises en sang blanc pour chaque coupe sont recalées identiques à la méthode choisie en sang noir. Il est possible de choisir un algorithme différent pour le traitement des images en sang noir et en sang blanc, mais il est préférable de choisir le même algorithme par facilité d'implémentation. Le recalage améliore sensiblement l'image qui sera fusionnée issue d'une pluralité d'images acquises dans une même coupe, car le contraste est meilleur et les artefacts de la respiration ont pu être compensés grâce à l'algorithme non rigide.

Selon un premier exemple, un algorithme non rigide est un algorithme basé sur la méthode de l'information mutuelle entre images fondée sur des relations statistiques. La fonction à optimiser peut-être mise en œuvre par un critère de similarité statistique. Un intérêt de cette méthode est que l'appariement entre attributs homologues d'images d'une même coupe est indépendant de leur position géométrique.

Selon un second exemple, un algorithme non rigide basé sur un modèle de transformation est mis en œuvre. Le modèle de transformation permet de déterminer des fonctions permettant de minimiser l'écart entre deux images. L'écart peut être traduit par une erreur géométrique à minimiser. Différentes approches peuvent être utilisées telles que celles se basant sur l'extraction à partir de chacune des images de primitives géométriques ou de descripteurs de formes tels que des points saillants, des singularités de formes ou de contours. Une approche paramétrique ou non paramétrique peut être utilisée.

Selon un exemple d'optimisation d'un modèle de transformation ou d'un critère de similarité, la méthode des moindres carrés peut être utilisée.

D'autres méthodes d'optimisation peuvent être mises en œuvre telles que la descente de gradients. Toutefois, cette dernière méthode s'applique plus particulièrement sur les intensités d'images et n'est pas optimale dans le cadre de l'invention puisqu'on cherche à optimiser la netteté et le contraste de l'image fusionnée. Néanmoins, l'invention inclut ce mode de réalisation.

Le recalage peut être réalisé en choisissant une image de référence et en déterminant une fonction de transformation des autres images de la même coupe vis-à-vis de cette image. Chaque image est alors recalée en optimisant une transformation pour obtenir l'image de référence selon un critère géométrique à partir de l'image considérée.

Lorsqu'une pluralité d'images sont recalées entre elles dans un plan de coupe, il est possible d'opérer des opérations visant à fusionner ces images afin de produire une unique image fusionnée par plan de coupe.

Selon un mode de réalisation, une étape de moyennage des images d'une même coupe est opérée de manière à réduire le bruit et augmenter le rapport signal sur bruit.

Le moyennage présente l'avantage de conserver le détail de l'image, puisqu'il accroît le rapport signal sur bruit SNR. Cette technique permet de lisser le bruit pour réduire les artefacts résiduels de l'image. En outre, le moyennage permet d'améliorer la profondeur de bit de l'image numérique au-delà de ce qui est possible avec une image unique.

Un intérêt de l'étape de moyennage des images réalisées d'une même coupe est de réduire la déviation maximum. L'amplitude du bruit diminue comme la racine carrée du nombre d'images utilisées, c'est-à-dire qu'avec seulement 4 images, on peut réduire l'amplitude du bruit par un facteur deux. Selon un exemple d'une acquisition en respiration libre d'une durée de 2min, il est possible de recueillir 4 à 5 images par plan de coupe, ce qui permet d'obtenir de bonnes performances de réduction de bruit.

Chaque ensemble d'images acquises d'une part en sang noir et d'autre part en sang blanc sont respectivement fusionnées de manière à produire une image unique sang noir IM_{1F}^{k} et une image sang blanc IM_{2F}^{k} unique par plan de coupe PCₖ.

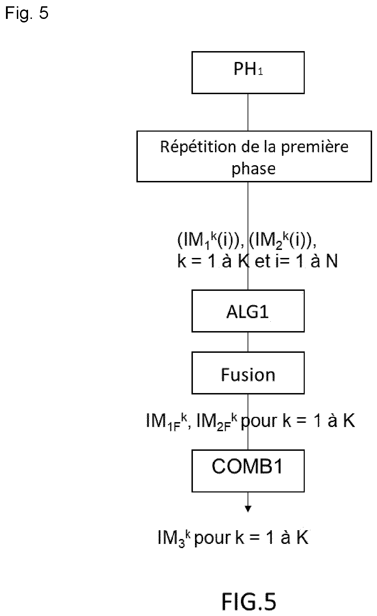

Autrement dit, on produit une unique image sang noir fusionnée IM_{1F}^{k} et une image sang blanc fusionnée IM_{2F}^{k} par plan de coupe PCₖ comme visible en figure 5.

Selon un mode de réalisation, le procédé de l'invention comprend alors une étape de colorisation des pixels les plus lumineux de l'image sang noir. Les pixels les plus lumineux correspondent notamment à la cicatrice compte tenu du fait que les signaux du muscle et du sang s'annulent et sont représentés par des pixels de faible intensité, c'est-à-dire noir. La colorisation de la cicatrice permet d'identifier et de représenter le contour de cette dernière.

Un intérêt de la colorisation est de permettre de clairement identifier la cicatrice et d'utiliser cette représentation combinée avec l'image en sang blanc dans laquelle les contours des muscles sont représentés. Ainsi, une telle combinaison permet d'identifier et de localiser la cicatrice notamment vis-à-vis du muscle et du sang à proximité de la cicatrice.

La combinaison COMB1 des images sang noir IM_{1F}^{k} et sang blanc IM_{2F}^{k} peut être réalisée pour chaque plan de coupe PCₖ. Ainsi, la même opération de combinaison permet d'aboutir à une pluralité d'images combinées sang noir - sang blanc pour produire une image unique IM₃^{k} par plan de coupe PCₖ.

La combinaison COMB1 des images peut être réalisée de différentes manières. Selon un premier exemple, les images sont superposées entre elles. Les superpositions peuvent comprendre la prise en compte d'une variation de l'opacité de l'image sang noir afin de visualiser correctement les contours de l'image sang blanc. L'opacité des images en sang noir peut être configurée pour avoir une valeur comprise entre 40% et 90%, par exemple 75%. Selon un second exemple, les pixels de la cicatrice qui ont une luminance supérieure à un seuil donné sont extraits pour être intégrés dans l'image sang blanc.

D'autres méthodes de combinaison d'images peuvent être utilisées afin de produire une image unique à partir de l'image sang noir et de l'image sang blanc par coupe.

Autrement dit, pour chaque plan de coupe PCₖ on produit une unique image combinée IM₃^{k} à partir de l'image sang noir fusionnée IM_{1F}^{k} et de l'image sang blanc fusionnée IM_{2F}^{k}**.**

Un intérêt de la méthode de l'invention est de générer une pluralité d'images reconstruites par plan de coupe à partir d'une image sang noir et d'une image sang blanc. Selon la taille du cœur, entre 8 et 20 plans de coupes peuvent être sélectionnés. Le procédé de l'invention permet d'afficher à un utilisateur, plus particulièrement un radiologue, une image reconstruite pour chaque plan de coupe. Chacune des images permet d'afficher la présence ou non d'une cicatrice en la localisant précisément par rapport à l'anatomie du cœur, au myocarde et aux volumes de sang avoisinant la zone imagée.

## Revendications

1. Procédé de reconstruction d'une image d'un cœur d'un patient à partir d'un dispositif d'imagerie par résonance magnétique (MRI₁) générant un champ magnétique (B₀) comprenant :
- Une première phase (PH₁) de génération d'images comportant :
▪ Acquisition (ACQ₀) de l'activité électrique (ECG₁) du cœur du patient ;
▪ Génération (GEN₁) d'un premier signal radiofréquence d'inversion (Rfᵢ) de 180° pour inverser une aimantation longitudinale de tissus d'une zone imagée, ledit premier signal radiofréquence d'inversion (Rfᵢ) étant généré entre deux complexes QRS de l'activité électrique acquise (ECG₁), dite première phase inter-battement (Tc₁) ;
▪ Génération (GEN₂) d'une première préparation d'aimantation (TAₚᵣₑₚ) comportant un ensemble de pulses ({pi}) consécutivement à la génération du premier signal radio fréquence d'inversion (Rfᵢ) dans la même première phase inter-battement (Tc₁) ;
▪ Acquisition (ACQ₁) d'une première image 2D (IM₁) par une mesure d'aimantation de la zone imagée, ladite acquisition (ACQ₁) étant réalisée après une première durée (D₁) prédéfinie consécutivement à la génération de la première préparation d'aimantation (Tₚᵣₑₚ), ladite acquisition (ACQ₁) étant synchronisée avec le rythme cardiaque (ECG₁) à un premier marqueur temporel (M₁) de la même phase inter-battement (Tc₁);
▪ Génération (GEN₃) d'une seconde préparation d'aimantation comportant un ensemble de pulses ({pi}) dans une seconde phase inter-battement (Tc₂) succédant à la première phase inter-battement (Tc₁) ;
▪ Acquisition (ACQ₂) d'une seconde image 2D (IM₂) par une mesure d'aimantation de la zone imagée, ladite acquisition (ACQ₂) étant réalisée après une seconde durée (D₂) prédéfinie consécutivement à la génération de la seconde préparation d'aimantation (TBₚᵣₑₚ), ladite acquisition (ACQ₂) étant synchronisée avec l'acquisition d'un rythme cardiaque (ECG₁) au même premier marqueur temporel (M₁) de la seconde phase inter-battement (Tc₂) que le premier marqueur (M₁) de la première phase inter-battement (Tc₁) ;
- Répétition de la première phase (PH1), pour générer un sous-ensemble de premières images et un sous-ensemble de secondes images de chaque plan de coupe (PCₖ) d'une pluralité de plans de coupes du cœur, dans des phases inter-battements (Tcik) succédant la première et la seconde phases inter-battements (Tc1, Tc2) ;
- Application d'un algorithme de recalage non rigide (ALG₁) à chaque sous-ensemble de premières images (IM₁^{k}(i)) d'un des plans de coupe (PCₖ) pour recaler lesdites premières images (IM₁^{k}(i)) du sous-ensemble de premières images entre elles ;
- Application d'un algorithme de recalage non rigide (ALG₁) à chaque sous-ensemble de secondes images (IM₂^{k}(i)) d'un des plans de coupe (PCₖ) pour recaler lesdites secondes images (IM₂^{k}(i)) du sous-ensemble de secondes images entre elles ;
- Fusion, pour chacun des plans de coupe, d'une part des premières images recalées entre elles pour produire une première image fusionnée (IM_{1F}^{k}) et d'autre part des secondes images recalées entre elles pour produire une seconde image fusionnée (IM_{2F}^{k}) ;
- Génération, pour lesdits plans de coupe, d'un ensemble de troisièmes images 2D, chaque troisième image (IM₃^{k}) correspondant à une première combinaison (COMB1) réalisée entre la première image fusionnée et la seconde image fusionnée produites pour un des plans de coupe,
la première durée (D₁) étant déterminée de sorte que les premières images acquises sont des images ayant un premier contraste permettant d'afficher des images en sang noir et la seconde durée (D₂) étant déterminée de sorte que les secondes images acquises sont des images ayant un second contraste permettant d'afficher des images en sang blanc.

2. Procédé selon la revendication 1, dans lequel l'étape de recalage est suivie des étapes de :
- Moyennage des premières images recalées pour produire une première image fusionnée (IM_{1F}^{k}) pour chaque plan de coupe (PCₖ) ;
- Moyennage des secondes images recalées pour produire une première image fusionnée (IM_{2F}^{k}) pour chaque plan de coupe (PCₖ).

3. Procédé selon l'une quelconque des revendications 1 à 2, dans lequel la première durée (D₁) est définie de façon que l'aimantation longitudinale du sang et celle du myocarde sain s'annulent au même instant TI lors de l'acquisition (ACQ₁) de la première image 2D.

4. Procédé selon l'une quelconque des revendications 1 à 3, dans lequel la détermination de la première durée (D₁) est automatiquement calculée à partir d'un procédé mis en œuvre par ordinateur d'un temps d'inversion optimal réalisé à partir d'un traitement d'au moins une image acquise à partir d'une préséquence d'IRM.

5. Procédé selon l'une quelconque des revendications 1 à 4, comprenant une étape de colorisation des pixels de chaque première image fusionnée (IM_{1F}^{k}) ayant une luminance supérieure à un seuil donné, l'étape de colorisation étant réalisée préalablement à la première combinaison (COMB1).

6. Procédé selon l'une quelconque des revendications 1 à 5, dans lequel la première combinaison (COMB1) est une superposition de la première image (IM_{1F}^{k}) sur la seconde image fusionnée (IM_{2F}^{k}).

7. Procédé selon l'une quelconque des revendications 1 à 6, dans lequel :
▪ chaque génération d'une nouvelle première image et d'une nouvelle seconde image dans un nouveau plan de coupe du cœur est réalisée après :
▪ l'ensemble des répétitions de la première phase (PH₁) pour générer une pluralité de premières images ({IM₁^{k}(i)}_{i[1}-_{N]}) et de secondes images ({IM₂^{k}(i)}_{i[1}-_{N]}) d'un précédent plan de coupe (PCₖ) du cœur.

8. Procédé selon l'une quelconque des revendications 1 à 7, comportant :
▪ un affichage d'un ensemble de premières images fusionnées (IM_{1F}^{k}) pour chaque plan de coupe (PCₖ) ;
▪ un affichage d'un ensemble de secondes images fusionnées (IM_{2F}^{k}) pour chaque plan de coupe (PCₖ) ;
▪ un affichage d'un ensemble de troisièmes images 2D pour chaque plan de coupe (PCₖ) ;
▪ chaque première image (IM_{1F}^{k}) d'un plan de coupe donné (PCₖ) étant affichée au voisinage immédiat de la seconde image et de la troisième image (IM_{3F}^{k}) du même plan de coupe donné (PCₖ).

9. Procédé selon l'une quelconque des revendications 1 à 8, dans lequel la première image fusionnée (IM_{1F}^{k}) pour chaque plan de coupe (PCₖ) correspond à une image d'une coupe du cœur en sang noir, la seconde image fusionnée (IM_{2F}^{k}) pour chaque plan de coupe (PCₖ) correspond à une image d'une coupe du cœur en sang blanc, la troisième image (IM_{3F}^{k}) pour chaque plan de coupe (PCₖ) correspondant à une image d'une coupe de l'organe sur laquelle une cicatrice le cas échéant est affichée en couleur.

10. Procédé de reconstruction d'une image selon l'une quelconque des revendications 1 à 9, dans lequel la première et la seconde préparation d'aimantation (TAprep, TBprep) sont des pondérations T1rho.

11. Procédé de reconstruction d'une image selon l'une quelconque des revendications 1 à 10, dans lequel la première préparation d'aimantation et/ou la seconde préparation d'aimantation (TAprep, TBprep) est une préparation de type T2prep ou une préparation en transfert d'aimantation (MT).

12. Procédé de reconstruction d'une image selon l'une quelconque des revendications 1 à 11, dans lequel la génération (GEN₃) d'une seconde préparation d'aimantation (TBprep) comportant un ensemble de pulses ({pi}) dans une phase inter-battement (Tc_{N}) est succédé à une étape de filtre des images acquises correspondant à des zones graisseuses de l'organe imagé.

13. Procédé de reconstruction d'une image selon l'une quelconque des revendications 1 à 12, dans lequel les premières images (IM₁) et les secondes images (IM₂) sont acquises successivement de manière synchronisée avec l'activité électrique du cœur entre deux complexes QRS pendant plusieurs minutes en respiration libre lors d'un unique examen.

14. Procédé de reconstruction d'une image selon l'une quelconque des revendications 1 à 13, dans lequel les premières images et les secondes images sont acquises sur un ensemble de 8 à 20 plans de coupe de l'organe, la phase de réitération des acquisitions d'une pluralité de premières images ({ M₁^{k}(i)}_{i[1}-_{N]}) et de secondes images ({IM₂^{k}(i)}_{i[1}-_{N]}) dans un même plan de coupe (PCₖ) permettant d'acquérir entre 2 et 10 images de premières images ({IM₁^{k}(i)}_{i[1}-_{N]}) et entre 2 et 10 images de secondes images ({IM₂^{k}(i)}_{i[1}-_{N]}) par plan de coupe.

15. Système d'imagerie par résonnance magnétique (S₁) comprenant un générateur de champ magnétique (GEN_B₀, GEN_GRAD) et un dispositif radiofréquence (DISPO_RF), un électrocardiographe (ELC₁) et un dispositif de traitement (K₁), un afficheur (IHM₁) pour afficher les images générées, ledit système étant configuré pour mettre en œuvre le procédé de l'une quelconque des revendications 1 à 14.

## Patentansprüche

1. Verfahren zur Bildrekonstruktion des Herzens eines Patienten aus einem Magnetresonanztomographiegerät (MRI₁), das ein Magnetfeld (B₀) generiert, umfassend:
- Eine erste Phase (PH₁) der Bildgenerierung, umfassend:
▪ Erfassung (ACQ₀ ) der elektrischen Aktivität (EKG₁) des Herzens des Patienten;
▪ Generierung (GEN₁) eines ersten 180°-Umkehr-Hochfrequenzsignals (Rfᵢ), um eine Längsmagnetisierung von Geweben eines abgebildeten Bereichs umzukehren, wobei das erste Umkehr-Hochfrequenzsignal (Rfᵢ) zwischen zwei QRS-Komplexen der erfassten elektrischen Aktivität (EKG₁), der sogenannten ersten Zwischenschlagphase (Tc₁), generiert wird;
▪ Generierung (GEN₂) einer ersten Magnetisierungsvorbereitung (TAₚᵣₑₚ) mit einer Reihe von Impulsen ({pi}) infolge der Generierung des ersten Umkehr-Hochfrequenzsignals (Rfᵢ) in derselben ersten Zwischenschlagphase (Tc₁);
▪ Erfassung (ACQ₁) eines ersten 2D-Bildes (IM₁) durch eine Magnetisierungsmessung des bildgebenden Bereichs, wobei die Erfassung (ACQ₁) nach einer ersten vordefinierten Dauer (D₁) infolge der Generierung der ersten Magnetisierungsvorbereitung (Tₚᵣₑₚ) durchgeführt wird, wobei die Erfassung (ACQ₁) mit dem Herzrhythmus (EKG₁) an einem ersten Zeitmarker (M₁) der gleichen Zwischenschlagphase (Tc₁) synchronisiert wird;
▪ Generierung (GEN₃) einer zweiten Magnetisierungsvorbereitung, die eine Reihe von Impulsen ({pi}) in einer zweiten Zwischenschlagphase (Tc₂) umfasst, die der ersten Zwischenschlagphase (Tc₁) folgt;
▪ Erfassung (ACQ₂) eines zweiten 2D-Bildes (IM₂) durch eine Magnetisierungsmessung des bildgebenden Bereichs, wobei die Erfassung (ACQ₂) nach einer zweiten vordefinierten Dauer (D₂) infolge der Generierung der zweiten Magnetisierungsvorbereitung (TB_{Vorb}) durchgeführt wird, wobei die Erfassung (ACQ₂) mit der Erfassung eines Herzrhythmus (EKG₁) am gleichen ersten Zeitmarker (M₁) der zweiten Zwischenschlagphase (Tc₂) synchronisiert wird, wie der erste Marker (M₁) der ersten Zwischenschlagphase (Tc₁);
- Wiederholung der ersten Phase (PH1), um eine Untergruppe erster Bilder und eine Untergruppe zweiter Bilder jeder Schnittebene (PCₖ) einer Vielzahl von Schnittebenen des Herzens in den den ersten und den zweiten Schlagphasen (Tc1, Tc2) folgenden Schlagphasen (Tcik) zu generieren;
- Anwendung eines nicht starren Ausrichtungsalgorithmus (ALG₁) auf jede Untergruppe erster Bilder (IM₁^{k}(i)) einer der Schnittebenen (PCₖ) zum Ausrichten dieser ersten Bilder (IM₁^{k}(i)) der Untergruppe erster Bilder untereinander;
- Anwendung eines nicht starren Ausrichtungsalgorithmus (ALG₁) auf jede Untergruppe zweiter Bilder (IM₂^{k}(i)) einer der Schnittebenen (PCₖ) zum Ausrichten dieser zweiten Bilder (IM₂^{k}(i)) der Untergruppe zweiter Bilder untereinander;
- Zusammenführung, für jede der Schnittebenen, einerseits der ersten untereinander ausgerichteten Bilder, um ein erstes zusammengeführtes Bild zu generieren (IM_{1F}^{k}) und andererseits der zweiten untereinander ausgerichteten Bilder, um ein zweites zusammengeführtes Bild zu generieren (IM_{2F}^{k});
- Generierung, für die Schnittebenen, einer Reihe von dritten 2D-Bildern, wobei jedes dritte Bild (IM₃^{k}) einer ersten Kombination (COMB1) aus dem ersten zusammengeführten Bild und dem zweiten zusammengeführten Bild, die für eine der Schnittebenen generiert wurden, entspricht,
wobei die erste Dauer (D₁) so bestimmt ist, dass die ersten erfassten Bilder Bilder mit einem ersten Kontrast sind, um Schwarzblutbilder anzeigen zu können, und die zweite Dauer (D₂) so bestimmt ist, dass die zweiten erfassten Bilder Bilder mit einem zweiten Kontrast sind, um Hellblutbilder anzeigen zu können.

2. Verfahren nach Anspruch 1, wobei dem Ausrichtungsschritt folgende Schritte folgen:
- Mittelwertbildung der ersten ausgerichteten Bilder, um ein erstes zusammengeführtes Bild (IM_{1F}^{k}) für jede Schnittebene (PCₖ) zu generieren;
- Mittelwertbildung der zweiten ausgerichteten Bilder, um ein erstes zusammengeführtes Bild (IM_{2F}^{k}) für jede Schnittebene (PCₖ ) zu generieren.

3. Verfahren nach einem der Ansprüche 1 bis 2, wobei die erste Dauer (D₁) so definiert ist, dass die Längsmagnetisierung des Blutes und jene des gesunden Herzmuskels zum gleichen Zeitpunkt TI bei der Erfassung (ACQ₁) des ersten 2D-Bildes aufgehoben werden.

4. Verfahren nach einem der Ansprüche 1 bis 3, wobei die Bestimmung der ersten Dauer (D₁) automatisch aus einem computergestützten Verfahren einer optimalen Umkehrzeit berechnet wird, die aus einer Verarbeitung von mindestens einem aus einer MRT-Präsequenz erfassten Bild durchgeführt wird.

5. Verfahren nach einem der Ansprüche 1 bis 4, umfassend einen Schritt zum Färben der Pixel jedes ersten zusammengeführten Bildes (IM_{1F} ^{k}) mit einer Leuchtdichte über einem bestimmten Schwellenwert, wobei der Färbeschritt vor der ersten Kombination (COMB1) durchgeführt wird.

6. Verfahren nach einem der Ansprüche 1 bis 5, wobei die erste Kombination (COMB1) eine Überlagerung des ersten Bildes (IM_{1F}^{k}) über dem zweiten zusammengeführten Bild (IM_{2F}^{k}) ist.

7. Verfahren nach einem der Ansprüche 1 bis 6, wobei:
▪ Jede Generierung eines neuen ersten Bildes und eines neuen zweiten Bildes in einer neuen Schnittebene des Herzens erfolgt nach:
▪ allen Wiederholungen der ersten Phase (PH₁), um mehrere erste Bilder ({IM₁^{k}(i)}_{i[1}-_{N]}) und zweite Bilder ({IM₂^{k}(i)}_{i[1}-_{N]}) einer vorhergehenden Schnittebene (PCₖ) des Herzens zu generieren.

8. Verfahren nach einem der Ansprüche 1 bis 7, umfassend:
▪ eine Anzeige einer Reihe von ersten zusammengeführten Bildern (IM_{1F}^{k}) für jede Schnittebene (PCₖ);
▪ eine Anzeige einer Reihe von zweiten zusammengeführten Bildern (IM_{2F}^{k}) für jede Schnittebene (PCₖ);
▪ eine Anzeige einer Reihe von dritten 2D-Bildern für jede Schnittebene (PCₖ);
▪ wobei jedes erste Bild (IM_{1F}^{k}) einer bestimmten Schnittebene (PCₖ) in unmittelbarer Nähe des zweiten Bildes und des dritten Bildes (IM_{3F}^{k}) derselben bestimmten Schnittebene (PCₖ) angezeigt wird.

9. Verfahren nach einem der Ansprüche 1 bis 8, wobei das erste zusammengeführte Bild (IM_{1F}^{k}) für jede Schnittebene (PCₖ) einem Schwarzblutbild eines Schnitts des Herzens entspricht, das zweite zusammengeführte Bild (IM_{2F}^{k}) für jede Schnittebene (PCₖ) einem Hellblutbild eines Schnitts des Herzens entspricht, das dritte Bild (IM_{3F}^{k}) für jede Schnittebene (Pck)ₖ) einem Bild eines Organschnitts entspricht, auf dem eine etwaige Narbe farblich dargestellt ist.

10. Verfahren zur Bildrekonstruktion nach einem der Ansprüche 1 bis 9, wobei die erste und zweite Magnetisierungsvorbereitung (TAprep, TBprep) T1rho-Gewichtungen sind.

11. Verfahren zur Bildrekonstruktion nach einem der Ansprüche 1 bis 10, wobei die erste Magnetisierungsvorbereitung und/oder die zweite Magnetisierungsvorbereitung (TAprep, TBprep) eine Vorbereitung vom Typ T2prep oder eine Magnetisierungstransfer-Vorbereitung (MT) ist.

12. Verfahren zur Bildrekonstruktion nach einem der Ansprüche 1 bis 11, wobei die Generierung (GEN₃) einer zweiten Magnetisierungsvorbereitung (TBprep) mit einer Reihe von Impulsen ({pi}) in einer Zwischenschlagphase (Tc_{N}) einem Schritt folgt, in dem die erfassten Bilder entsprechend fettigen Bereichen des abgebildeten Organs gefiltert werden.

13. Verfahren zur Bildrekonstruktion nach einem der Ansprüche 1 bis 12, wobei die ersten Bilder (IM₁) und die zweiten Bilder (IM₂) nacheinander synchron mit der elektrischen Aktivität des Herzens zwischen zwei QRS-Komplexen für mehrere Minuten bei freier Atmung während einer einzigen Untersuchung erfasst werden.

14. Verfahren zur Bildrekonstruktion nach einem der Ansprüche 1 bis 13, wobei die ersten Bilder und die zweiten Bilder in einer Reihe von 8 bis 20 Schnittebenen des Organs erfasst werden, wobei die Phase der Wiederholung der Erfassungen mehrerer erster Bilder (IM₁^{k}(i)}_{i[1}-_{N]}) und zweiter Bilder (lM₂^{k}(i)}_{i[1}-_{N]}) in derselben Schnittebene (PCₖ) die Erfassung von 2 bis 10 Bildern erster Bilder ({IM₁^{k}(i)}_{i[1}-_{N]}) und von 2 bis 10 Bildern zweiter Bilder ({IM₂^{k}(i)}_{i[1}-_{N]}) pro Schnittebene ermöglicht.

15. Magnetresonanztomographiesystem (S₁), umfassend einen Magnetfeldgenerator (GEN_B₀, GEN_GRAD) und eine Hochfrequenzvorrichtung (DISPO_RF), einen Elektrokardiographen (ELC₁) und eine Verarbeitungsvorrichtung (K₁), ein Display (MMI₁) zur Anzeige der generierten Bilder, wobei das System so konfiguriert ist, dass es das Verfahren nach einem der Ansprüche 1 bis 14 implementiert.

## Claims

1. Method for reconstructing an image of a patient's heart from a magnetic resonance imaging device (MRI₁) generating a magnetic field (B₀) comprising:
- A first phase (PH₁) of image generation including:
▪ Acquisition (ACQ₀) of the electrical activity (ECG₁) of the patient's heart;
▪ Generation (GEN₁) of a first 180° inversion radio frequency signal (Rfᵢ) to inverse a longitudinal magnetization of tissues of an imaged area, said first inversion radio frequency signal (Rfᵢ) being generated between two QRS complexes of the acquired electrical activity (ECG₁), so-called first inter-beat phase (Tc₁);
▪ Generation (GEN₂) of a first magnetization preparation (TAₚᵣₑₚ) including a set of pulses ({pi}) following the generation of the first inversion radio frequency signal (Rfᵢ) in the same first inter-beat phase (Tc₁);
▪ Acquisition (ACQ₁) of a first 2D image (IM₁) by a magnetization measurement of the imaged area, said acquisition (ACQ₁) being performed after a first predefined duration (D₁) following the generation of the first magnetization preparation (Tₚᵣₑₚ), said acquisition (ACQ₁) being synchronized with the heart rhythm (ECG₁) at a first time marker (M₁) of the same inter-beat phase (Tc₁);
▪ Generation (GEN₃) of a second magnetization preparation including a set of pulses ({pi}) in a second inter-beat phase (Tc₂) following the first inter-beat phase (Tc₁);
▪ Acquisition (ACQ₂) of a second 2D image (IM₂) by a magnetization measurement of the imaged area, said acquisition (ACQ₂) being performed after a second predefined duration (D₂) following the generation of the second magnetization preparation (TBₚᵣₑₚ), said acquisition (ACQ₂) being synchronized with the acquisition of a heart rhythm (ECG₁) at the same first time marker (M₁) of the second inter-beat phase (Tc₂) as the first marker (M₁) of the first inter-beat phase (Tc₁);
- Repetition of the first phase (PH1), to generate a subset of first images and a subset of second images of each section plane (PCₖ) of a plurality of section planes of the heart, in inter-beat phases (Tcik) following the first and second inter-beat phases (Tc₁, Tc₂);
- Application of a non-rigid registration algorithm (ALG₁) to each subset of first images (IM₁^{k}(i)) of one of the section planes (PCₖ) to register said first images (IM₁^{k}(i)) of the subset of first images together;
- Application of a non-rigid registration algorithm (ALG₁) to each subset of second images (IM₂^{k}(i)) of one of the section planes (PCₖ) to register said second images (IM₂^{k}(i)) of the subset of second images together;
- Merging, for each of the section planes, on the one hand the first images registered together to produce a first merged image (IM_{1F}^{k}) and on the other hand the second images registered together to produce a second merged image (IM_{2F}^{k});
- Generation, for said section planes, of a set of third 2D images, each third image (IM₃^{k}) corresponding to a first combination (COMB1) made between the first merged image and the second merged image produced for one of the section planes, the first duration (D₁) being determined such that the first acquired images are images having a first contrast making it possible to display black blood images and the second duration (D₂) being determined such that the second acquired images are images having a second contrast making it possible to display bright blood images.

2. Method according to claim 1, wherein the registration step is followed by the steps of:
- Averaging the first registered images to produce a first merged image (IM_{1F}^{k}) for each section plane (PCₖ);
- Averaging the second registered images to produce a first merged image (IM_{2F}^{k}) for each section plane (PCₖ).

3. Method according to any one of claims 1 to 2, wherein the first duration (D₁) is defined in such a way that the longitudinal magnetization of blood and that of healthy myocardium are nulled at the same instant TI of the acquisition (ACQ₁) of the first 2D image.

4. Method according to any one of claims 1 to 3, wherein the determination of the first duration (D₁) is automatically calculated from a computer-implemented method of an optimal inversion time performed from a processing of at least one image acquired from an MRI pre-sequence.

5. Method according to any one of claims 1 to 4, comprising a step of coloring the pixels of each first merged image (IM_{1F}^{k}) having a luminance greater than a given threshold, the coloring step being performed prior to the first combination (COMB1).

6. Method according to any one of claims 1 to 5, wherein the first combination (COMB1) is an overlay of the first image (IM_{1F}^{k}) on the second merged image (IM_{2F}^{k}).

7. Method according to any one of claims 1 to 6, wherein:
▪ each generation of a new first image and a new second image in a new heart section plane is performed after:
▪ all the repetitions of the first phase (PH₁) to generate a plurality of first images ({IM₁^{k}(i)}_{i[1}-_{N]}) and second images ({IM₂^{k}(i)}_{i[1}-_{N]}) of a preceding section plane (PCₖ) of the heart.

8. Method according to any of claims 1 to 7, comprising:
▪ a display of a set of first merged images (IM_{1F}^{k}) for each section plane (PCₖ);
▪ a display of a set of second merged images (IM_{2F}^{k}) for each section plane (PCₖ);
▪ a display of a set of third 2D images for each section plane (PCₖ);
▪ each first image (IM_{1F}^{k}) of a given section plane (PCₖ) being displayed in the immediate vicinity of the second image and the third image (IM_{3F}^{k}) of the same given section plane (PCₖ).

9. Method according to any one of claims 1 to 8, wherein the first merged image (IM_{1F}^{k})for each section plane (PCₖ) corresponds to a black blood image of a section of the heart, the second merged image (IM_{2F}^{k}) for each section plane (PCₖ) corresponds to a bright blood image of a section of the heart, the third image (IM_{3F}^{k}) for each section plane (PCₖ) corresponding to an image of a section of the organ on which a scar, if present, is displayed in color.

10. Method for reconstructing an image according to any one of claims 1 to 9, wherein the first and second magnetization preparation (TAprep, TBprep) are T1rho weightings.

11. Method for reconstructing an image according to any one of claims 1 to 10, wherein the first magnetization preparation and/or the second magnetization preparation (TAprep, TBprep) is a preparation of the T2prep type or a magnetization transfer preparation (MT).

12. Method for reconstructing an image according to any one of claims 1 to 11, wherein the generation (GEN₃) of a second magnetization preparation (TBprep) including a set of pulses ({pi}) in an inter-beat phase (Tc_{N}) is followed by a step of filtering the acquired images corresponding to fatty areas of the imaged organ.

13. Method for reconstructing an image according to any one of claims 1 to 12, wherein the first images (IM₁) and the second images (IM₂) are acquired successively in a manner synchronized with the electrical activity of the heart between two QRS complexes over a plurality of minutes in free breathing during a single examination.

14. Method for reconstructing an image according to any one of claims 1 to 13, wherein the first images and the second images are acquired on a set of 8 to 20 section planes of the organ, the phase of reiteration of the acquisitions of a plurality of first images ({M₁^{k}(i)}_{i[1}-_{N]}) and second images ({IM₂^{k}(i)}_{i[1}-_{N]}) in the same section plane (PCₖ) making it possible to acquire between 2 and 10 images of first images ({IM₁^{k}(i)}_{i[1}-_{N]}) and between 2 and 10 images of second images ({IM₂^{k}(i)}_{i[1}-_{N]}) per section plane.

15. Magnetic resonance imaging system (S₁) comprising a magnetic field generator (GEN_B₀, GEN_GRAD) and a radio frequency device (DISPO_RF), an electrocardiograph (ELC₁) and a processing device (K₁), a display (IHM₁) for displaying the generated images, said system being configured to implement the method of any one of claims 1 to 14.
